# EUROPEAN PATENT APPLICATION

(11) **EP 3 373 305 A1**
(43) Date of publication of application: **12.09.2018**
(21) Application number: 18160697.1
(22) Date of filing: 08.03.2018
(51) Int. Cl.: G16H 20/30

(54) **TRAINING DEVICE USABLE FOR REHABILITATION AND COMPUTER PROGRAM FOR TRAINING DEVICE USABLE FOR REHABILITATION**

(30) Priority: 10.03.2017 JP 2017046149
(71) Applicant: Seiko Epson Corporation, Tokyo 160-8801 (JP)
(72) Inventor: TANAKA, Hideki, Nagano, 392-8502 (JP); MARUYAMA, Yuya, Nagano, 392-8502 (JP); KITAZAWA, Takayuki, Nagano, 392-8502 (JP); OOUCHIDA, Yutaka, Miyagi, 980-8577 (JP); IZUMI, Shinichi, Miyagi, 980-8577 (JP); SATO, Yosuke, Miyagi, 980-8577 (JP)
(74) Representative: Hoffmann Eitle

(57) **Abstract**

A rehabilitation training device includes a motion parameter value acquirer that acquires a first motion parameter value relating to a first motion of a body of a user who uses the training device, a training image generator that generates N (an integer greater than or equal to 1) training images including a training image having a motion parameter value representing motion characteristics different from the first motion parameter value with respect to the first motion, based on the first motion parameter value, and a display that displays one training image of the N training images as a first training image.

## Description

### BACKGROUND

### 1. Technical Field

The present invention relates to a rehabilitation training device and a computer program for the rehabilitation training device.

### 2. Related Art

The training device described in JP-A-2007-20835 measures and analyzes a brain function of a patient (subject of motion) undergoing rehabilitation, objectively displays a relationship between a sensed subject of motion and rehabilitation effects, and selects a more suitable rehabilitation training, based on the results.

However, since an actual amount of exercise of the patient cannot be measured from brain activity, the brain activity is not sufficient as an index of exercise selection. Therefore, there is a problem that it is difficult to obtain sufficient training effects by visual stimulation.

### SUMMARY

An advantage of some aspects of the invention is to solve at least a part of the problems described above, and the invention can be implemented in the following forms or application examples.
(1) According to an aspect of the invention, a training device usable for rehabilitation is provided. The training device includes a motion parameter value acquirer (a motion parameter sensor) that acquires a first motion parameter value relating to a first motion of a body part of a user who uses the training device, a training image generator (an image generating circuit) that generates a training image having a motion parameter value representing motion characteristics different from the first motion parameter value with respect to the first motion, based on the first motion parameter value, and a display that displays the training image.

According to this aspect, a motion parameter value relating to a first motion of a user who uses the training device is acquired, a training image representing motion characteristics different from the first motion parameter value with respect to the first motion is generated, based on the motion parameter value, and the user can do motion training by viewing the training image, and thus, the user can do motion training by using an appropriate training image.

The training image generator may generate N (an integer greater than or equal to 2) training images including the training image having the motion parameter value representing the motion characteristics different from the first motion parameter value with respect to the first motion, based on the first motion parameter value, and the display may display a first training image that is selected from the N training images.

With this configuration, a motion parameter value with respect to a first motion of a user who uses a training device is acquired and N training images are generated based on the motion parameter value, and the user can do motion training by viewing a first training image selected from the N training images, and thus, the user can do motion training by using an appropriate first training image.

The display may be a transmissive display through which the user visually recognizes the body of the user while visually recognizing the first training image that is displayed on the display.

With this configuration, a user can visually recognize a body of the user who does motion training while viewing a first training image, and thus, the user can obtain sufficient training effects by visual stimulation.

The training device may further include an image-capturer (a video camera) that captures an image of a body motion of the user, and the display may be an immersion display that displays an image of the body motion of the user that is captured by the image-capturer in real time together with the first training image.

With this configuration, a user can view an image of a body motion of the user who does motion training displayed on a display captured by an image-capturer in real time, while viewing a first training image displayed on the display, and thus, it is possible to obtain sufficient training effects by visual stimulation.

The motion parameter value acquirer may acquire a second motion parameter value relating to a second motion when the user does motion training, based on the first training image. The training image generator may select a second training image from among the N training images, based on the second motion parameter value. The display may display the second training image.

With this configuration, a second training image suitable for motion training of a user can be selected from among N generated training images and can be displayed on a display as the second training image.

The first motion parameter value and the second motion parameter value may be moving speeds of a specific joint of the user.

The first motion parameter value may be one of a moving speed and a moving angle of a specific joint of the user, and the second motion parameter value may be the other one of the moving speed and the moving angle of the specific joint.

With this configuration, a moving speed of a specific joint and a moving angle of the specific joint can be used as a first motion parameter value and a second motion parameter value.

The training device may further include at least one of an image-capturer that captures an image of a body of the user, a goniometer capable of measuring an angle of a specific joint of the user, and an acceleration sensor capable of measuring acceleration when the specific joint moves, and the motion parameter value acquirer may acquire the first motion parameter value and the second motion parameter value by using at least one of the outputs of the image-capturer, the goniometer, and the acceleration sensor.

With this configuration, a motion parameter value acquirer can acquire a first motion parameter value and a second motion parameter value by using a signal from a goniometer or an acceleration sensor in addition to an image from an image-capturer.

According to another aspect of the invention, a controller of a training device usable for rehabilitation is provided. The controller includes a motion parameter value acquirer that acquires a first motion parameter value relating to a first motion of a body of a user who uses the training device, a training image generator that generates N (an integer greater than or equal to 2) training images including a training image having a motion parameter value representing motion characteristics different from the first motion parameter value with respect to the first motion, based on the first motion parameter value, and a transmitter that outputs one of the N training images to a display as a first training image.

According to this aspect, a motion parameter value relating to a first motion of a user who uses a training device is acquired, N training images are generated based on the motion parameter value, and a user can do motion training by viewing a first training image selected from the N training images, and thus, the user can do the motion training by using an appropriate first training image.

According to still another aspect of the invention, a training device usable for rehabilitation is provided. The training device includes a memory that stores in advance N training images having motion parameter values different from each other with respect to a first motion of a body of a user who uses the training device, among N (an integer greater than or equal to 2) training images, and a display that displays a first training image which is selected from the N training images.

According to this aspect, a user can do motion training by viewing a first training image selected from N training images, the user can do motion training by using an appropriate first training image.

The invention can be realized in various forms. For example, the invention can be realized as a computer program for a training device usable for rehabilitation, in addition to a training device usable for rehabilitation.

### BRIEF DESCRIPTION OF THE DRAWINGS

Embodiments of the invention will be described with reference to the accompanying drawings, wherein like numbers reference like elements.
Fig. 1 is an explanatory view illustrating a training device usable for rehabilitation according to a first embodiment.
Fig. 2 is an explanatory diagram illustrating a controller of the training device.
Fig. 3 is a processing flowchart of the training device.
Fig. 4 is an explanatory view schematically illustrating some of the processing steps of the processing flowchart.
Fig. 5 is an explanatory view illustrating a state of a specific part viewed by a user through a display.
Fig. 6 is an explanatory view illustrating a state of the specific part viewed by the user through the display.
Fig. 7 is an explanatory view illustrating a state of the specific part viewed by the user through the display.
Fig. 8 is an explanatory view illustrating a state of the specific part viewed by the user through the display.
Fig. 9 is an explanatory view illustrating a state of the specific part viewed by the user through the display.
Fig. 10 is an explanatory view illustrating a state of the specific part viewed by the user through the display.
Fig. 11 is an explanatory view illustrating a state of the specific part viewed by the user through the display.
Fig. 12 is a processing flowchart of a training device according to a second embodiment.
Fig. 13 is an explanatory view schematically illustrating some of the processing steps of the processing flowchart of the training device according to the second embodiment.
Fig. 14 is a processing flowchart of a training device according to a third embodiment.
Fig. 15 is an explanatory view schematically illustrating a part of a second half of the processing steps of a processing flowchart of a training device according to a third embodiment.
Fig. 16 is a processing flowchart of a training device according to a fourth embodiment.
Fig. 17 is a processing flowchart of a training device according to a fifth embodiment.
Fig. 18 is an explanatory view illustrating a training device according to Modification Example 1.
Fig. 19 is an explanatory diagram illustrating a controller according to Modification Example 2.
Fig. 20 is an explanatory view illustrating a training device according to Modification Example 3.
Fig. 21 is an explanatory view illustrating a training device according to Modification Example 4.

### DESCRIPTION OF EXEMPLARY EMBODIMENTS

### First Embodiment

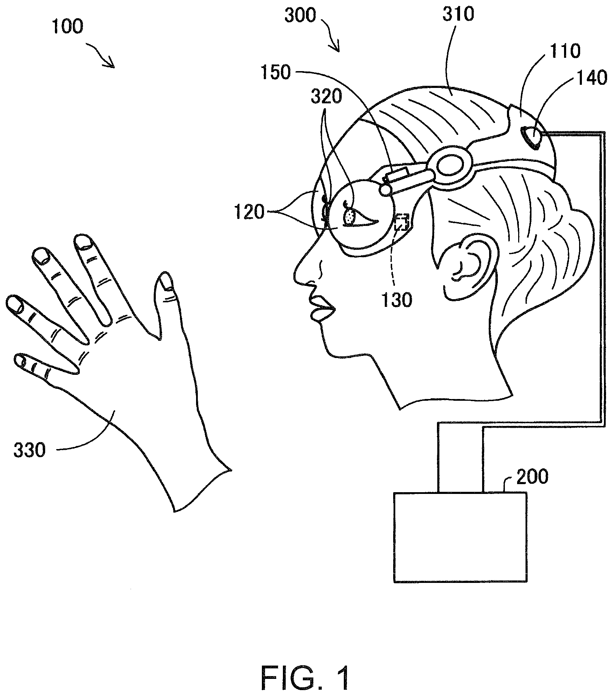

Fig. 1 is an explanatory view illustrating a training device 100 usable for rehabilitation according to a first embodiment. The training device 100 includes a head mounted display 110 and a controller 200. The head mounted display 110 is mounted on the head 310 of a user 300 of the training device 100. The head mounted display 110 includes a display 120, a projector 130, a communication interface 140, and an image-capturer (e.g., camera) 150. In the present embodiment, the display 120 is provided at a position facing eyes 320 of the user 300. The projector 130 projects light onto the display 120. The display 120 reflects light projected from the projector 130 toward the eyes 320 of the user 300. The user 300 can view a virtual image by the reflected light from the display 120. In addition, the display 120 is a transmissive display so it not only can reflect light from the projector 130, but can also transmit light from a side opposite to the projector 130. Therefore, the user 300 can view the hand 330, which is a specific part (body part), through the display 120 together with an image projected from the projector 130 onto the display 120. The head mounted display 110 is provided with two displays 120, one for the right eye and one for the left eye, and also, two projectors 130, one for the right eye and one for the left eye. The two projectors 130 may display the same image on the two displays 120 or may display different images, respectively. In addition, the projector 130 may project an image for the right eye and an image for the left eye on the two displays 120, respectively, such that the user 300 views a stereoscopic image.

The image-capturer 150 captures an image of a specific part (for example, a hand or a foot) of the user 300. In the training device 100 according to the first embodiment, the head mounted display 110 includes the image-capturer 150, but the image-capturer 150 may be provided separately from the head mounted display 110. However, it is preferable that an image of the specific part captured by the image-capturer 150 is similar in appearance to the specific part that the user 300 views through the display 120. Thus, it is preferable that the image-capturer 150 is provided at a position close to the eyes 320 of the user 300 on the head mounted display 110. The image-capturer 150 may be a stereo camera capable of capturing a stereoscopic image or may be a monaural camera.

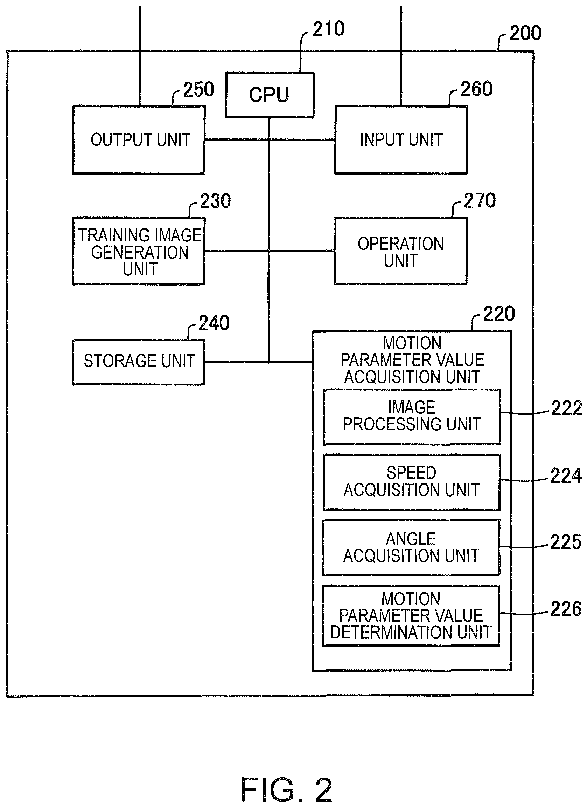

Fig. 2 is an explanatory diagram illustrating the controller 200 of the training device 100. The controller 200 includes a CPU 210, a motion parameter value acquirer 220, a training image generator 230, a memory 240, a transmitter 250, a receiver 260, and an operation unit (e.g., user interface) 270. In the first embodiment, the motion parameter value acquirer 220 acquires a motion parameter value from the image captured by the image-capturer 150. The motion parameter value acquirer 220 includes an image processor 222, a speed acquirer (e.g., sensor) 224, an angle acquirer (e.g., sensor) 225, and a motion parameter value determiner (e.g., a calculator) 226. The image processor 222 analyzes the motion of a specific part of the user 300 from the image acquired by the image-capturer 150. The speed acquirer 224 acquires a moving speed of a joint of the specific part of the user 300, based on analysis results of the image processor 222. The moving speed indicates how fast the user 300 can bend or extend the joint of the specific part, and can be represented by velocity or angular velocity. For example, the user 300 repeatedly moves the joint of the specific part a plurality of times, and the maximum speed among the plurality of times is set as the moving speed. Alternatively, an average speed of the plurality of times may be set as the moving speed. The angle acquirer 225 acquires the moving angle of the joint of the specific part of the user 300, based on the analysis results of the image processor 222. The moving angle of the joint indicates how much the user can expand or contract the joint of the specific part from a predetermined state and is represented by an angle (degrees or radians). For example, the user 300 repeatedly moves the joint of the specific part a plurality of times, and the maximum angle among the plurality of times is set as the moving angle. Alternatively, an average angle of the plurality of times may be set as the moving angle. The angle acquirer 225 can also acquire the angular speed of the joint of the specific part. The motion parameter value determiner 226 determines a motion parameter value, based on the values acquired by the speed acquirer 224 or the angle acquirer 225. In the first embodiment, the motion parameter value determiner 226 uses the moving speed of the joint of the specific part as a motion parameter value. In this case, conditions (for example, the moving angle, training time, severity of injury of the user 300, the age of the user 300, and the like) other than the moving speed used as the motion parameter value are set to be constants, and the moving speed of the joint of the specific part is measured by making the user 300 move the joint of the specific part, and a predetermined motion parameter value is acquired. If images acquired by the image-capturer 150 are different, the motion parameter values are deemed to be different. Alternatively, the conditions (for example, the moving angle, the training time, the severity of the injury of the user 300, the age of the user 300, and the like) other than the moving speed are set to be constants, and each image is generated under the condition that only the moving speed of the joint of the specific part is changed. The respective images generated are compared, and if the images are different, output images are made different by the moving speed. As an example, one input image is generated, and an image in which a reproduction speed of the input image is made different is generated. Alternatively, a robot is used, and conditions other than a moving speed of a specific part of the robot may be set to be constants. Alternatively, a motion range of the specific part of the robot is not changed and only the speed may be changed. The same applies to the moving angle and the like considered as the motion parameter value. For example, in a case where the moving angle is used as the motion parameter value, the moving speed of the specific part of the robot is not changed and only the moving angle may be changed. One of the speed acquirer 224 and the angle acquirer 225 can be omitted.

The training image generator 230 generates a training image to be imitated when the user 300 does motion training. The training image generator 230 can generate N (an integer equal to or greater than 1) training images including a training image having a motion parameter value representing advanced motion characteristics higher than a first motion parameter value acquired by the motion parameter value acquirer 220, as the training image. It is preferable that N is an integer equal to or greater than 2. The "advanced motion characteristics" mean characteristics which are evaluated as a higher function with respect to motion thereof. For example, the higher the speed is, the more advanced motion characteristics the moving speed of the joint may represent, and the slower the speed is, the more advanced motion characteristics the moving speed of the joint may represent. In addition, the larger the angle is, the more advanced motion characteristics the moving angle of the joint may represent, and the smaller the angle is, the more advanced motion characteristics the moving angle of the joint may represent. The N training images may include one or more training images in which a motion parameter value is less than or equal to (speed or angular speed is lower than or equal to) the first motion parameter value in addition to one or more training images in which the motion parameter value is greater than (speed or angular speed is faster than) the first motion parameter value. The memory 240 stores the training images.

The transmitter 250 transmits an image to an external display device such as the head mounted display 110. The receiver 260 receives an image from the image-capturer 150. The receiver 260 may be configured to be able to receive a signal from a sensor other than the image-capturer 150. If the motion parameter value acquirer 220 is configured to acquire the first motion parameter value from the signal from the sensor other than the image-capturer 150, the image-capturer 150 can be omitted. The operation unit 270 is used for the user 300 or a coach of the user 300 to operate the controller 200. The coach of the user 300 coaches and advises the user 300 for the motion training, for example, a doctor or a physical clinician.

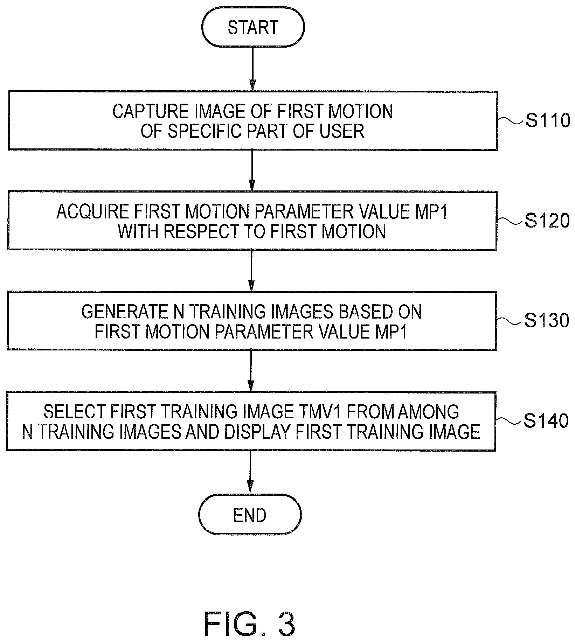

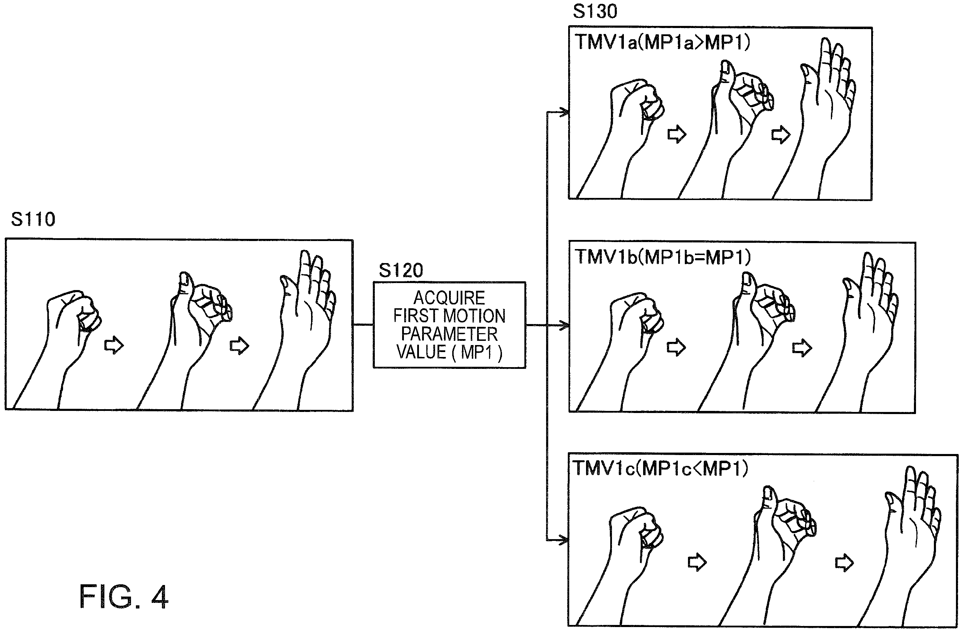

Fig. 3 is a processing flowchart of the training device 100. Fig. 4 is an explanatory view schematically illustrating some of the processing steps of the processing flowchart. In step S110, the image-capturer 150 captures an image of a first motion of a specific part of the user 300. In step S120, the motion parameter value determiner 226 determines a first motion parameter value MP1 relating to the first motion. In the first embodiment, a moving speed of a specific joint is used as the first motion parameter value MP1. For example, the motion parameter value determiner 226 determines the maximum value among the moving speeds of the joint performing first motions a plurality of times as the first motion parameter value MP1. An average value of the plurality of moving speeds of the joint may be set as the first motion parameter value MP1.

In step S130, the training image generator 230 generates N (an integer greater than or equal to 1) training images TMV1a, TMV1b, and TMV1c including a training image having a motion parameter value representing motion characteristics more advanced than the first motion parameter value MP1, based on the first motion parameter value MP1, and stores the training images in the memory 240. The N training images TMV1a, TMV1b, and TMV1c may be generated by using animation, or may be generated by modifying an image obtained in step S110. In the first embodiment, one training image TMV1b out of the N training images TMV1a, TMV1b, and TMV1c has the same motion parameter value as the first motion parameter value MP1 as the motion parameter value MP1b relating to a motion thereof, and the remaining training images TMV1a and TMV1c have a motion parameter value different from the first motion parameter value MP1 as the motion parameter values MP1a and MP1c relating to motions thereof. For example, the motion parameter value MP1a of the training image TMV1a is larger than the first motion parameter value MP1, and the motion parameter value MP1c of the training image TMV1c is smaller than the first motion parameter value MP1. It is preferable that the motion parameter value Mp1a of the training image TMV1a having advanced motion characteristics is, for example, 1.1 to 1.3 times the first motion parameter value MP1. The number N of the training images can be set to any integer greater than or equal to 1.

In step S140, the controller 200 selects one of the N training images as the first training image TMV1 and transmits the selected image to the head mounted display 110. The display 120 displays the first training image TMV1. For example, various methods described below can be adopted as a method of selecting the first training image TMV1 from among the N training images TMV1a, TMV1b, and TMV1c.
(a) The user 300 or a coach of the user 300 of the training device 100 selects a training image by using the operation unit 270.
(b) A training image is selected according to a predetermined selection rule (first selection rule). A specific example thereof is as follows.
   (b1) Among the training images in which a motion parameter value is larger than the first motion parameter value, the training image having the smallest motion parameter value is selected.
   (b2) The training image is randomly selected from the N training images.

In a case where N = 1, one training image generated in step S130 is displayed in step S140. In the present specification, a phrase "select the first training image from among the N training images" has a broad meaning also including this case.

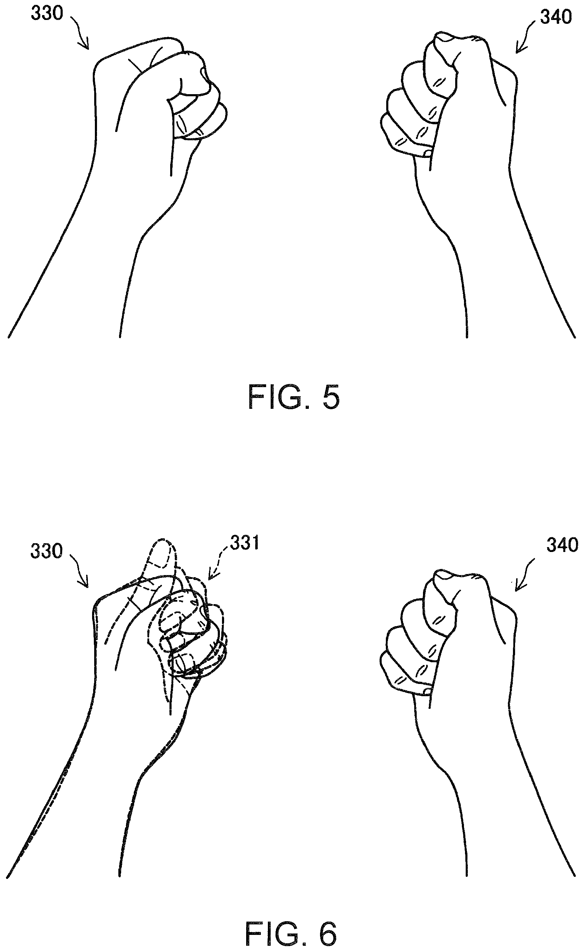

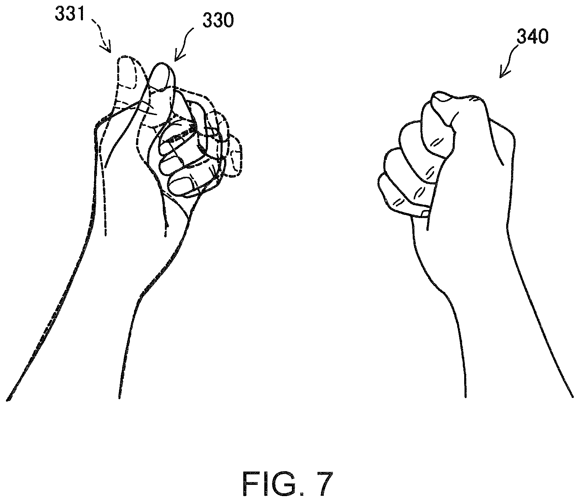

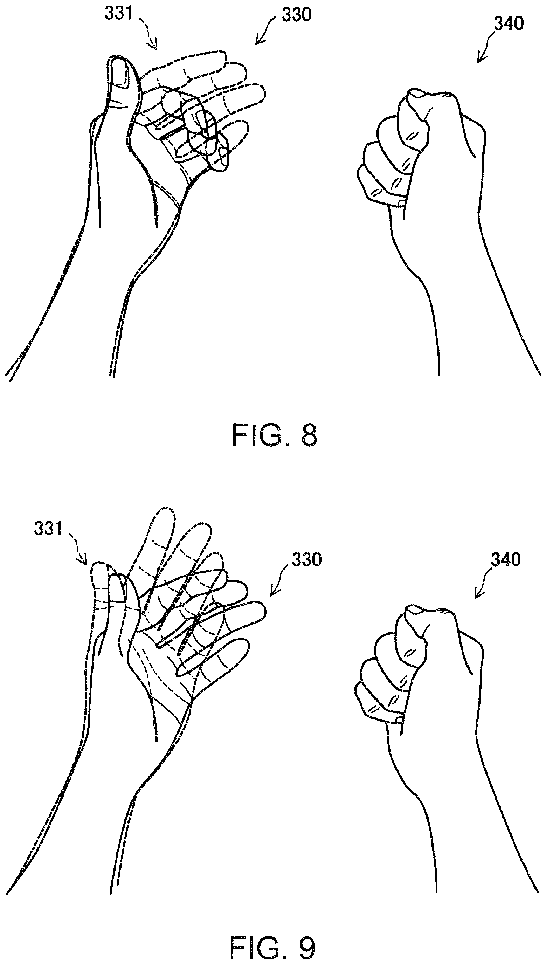

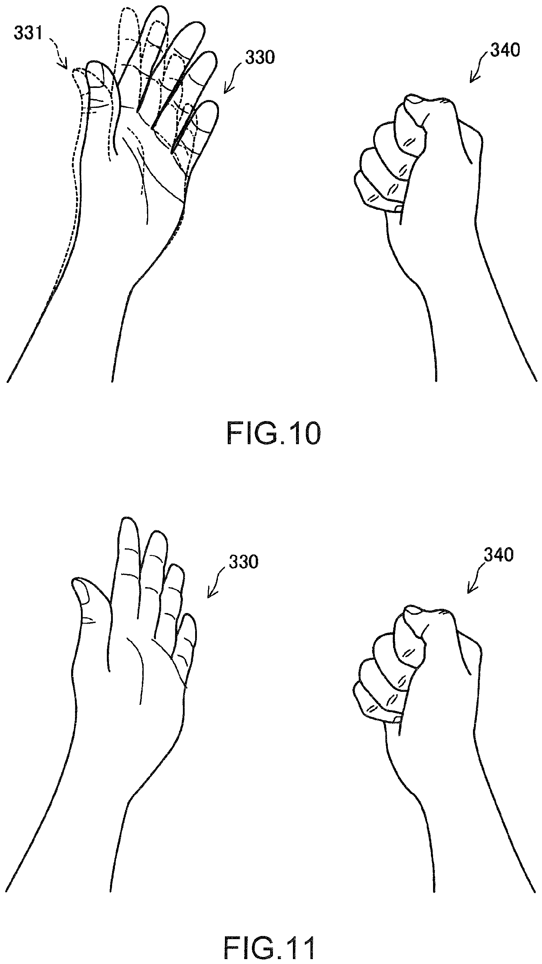

Figs. 5 to 11 are explanatory views illustrating states of the specific part viewed by the user 300 through the display 120. Fig. 5 illustrates a state where the hands 330 and 340 are grasped before one motion training starts, and Fig. 11 illustrates a state where the hand 330 is opened after the one motion training is completed. Figs. 6 to 10 sequentially illustrate intermediate states between Fig. 5 and Fig. 11. In addition, in Figs. 6 to 10, a solid line indicates a specific part (hand 330) viewed through the display 120, and a dashed line indicates an image 331 of the specific part (hand 330) transmitted from the controller 200 and the image 331 of the first training image TMV1. In Figs. 5 to 11, the other hand 340 which does not do motion training is also illustrated in addition to one hand 330 of the user 300 which does the motion training. As can be understood by comparing the solid line and the dashed line, the image 331 of the first training image TMV1 substantially overlaps the specific part (hand 330) viewed through the display 120, and the image 331 of the first training image TMV1 moves a little more than the specific part (hand 330) viewed through the display 120. That is, the user 300 does the motion training by moving the joint of the specific part so as to follow the image 331 of the first training image TMV1 indicated by a broken line. In Figs. 6 to 10, the solid line and the broken line substantially overlap each other, but the user 300 may do the motion training by moving the hand 330, for example, right and left or up and down, such that the image 331 of the first training image TMV1 does not overlap and is offset from the specific part viewed through the display 120.

According to the first embodiment, the first motion parameter value MP1 is acquired based on a first motion of the user 300, N (an integer greater than or equal to 1) training images TMV1a, TMV1b, and TMV1c including a training image having a motion parameter value representing more advanced motion characteristics than the first motion parameter value MP1 are generated, and the first training image TMV1 is selected from among the training images to be displayed, and thereby, the user 300 can do a motion training while viewing the image 331 of the appropriate first training image TMV1. In addition, since the user 300 can view both the image 331 of the first training image TMV1 and his/her motion when doing the motion training, it is possible to obtain sufficient training effects by visual stimulation and to do efficient motion training.

### Second Embodiment

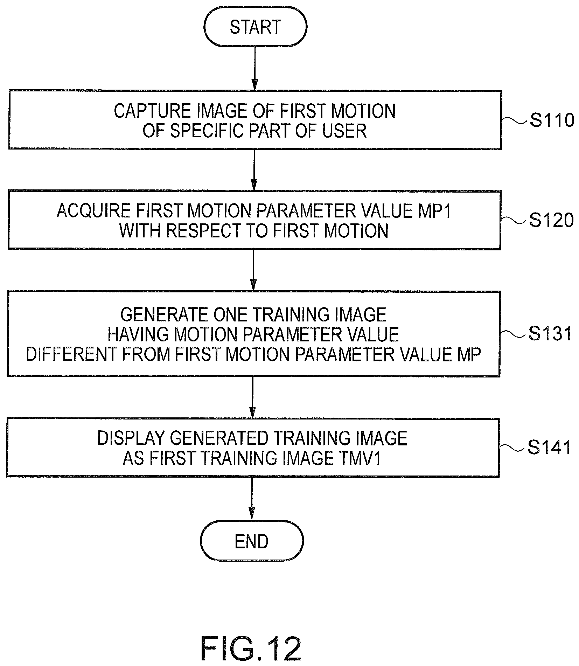

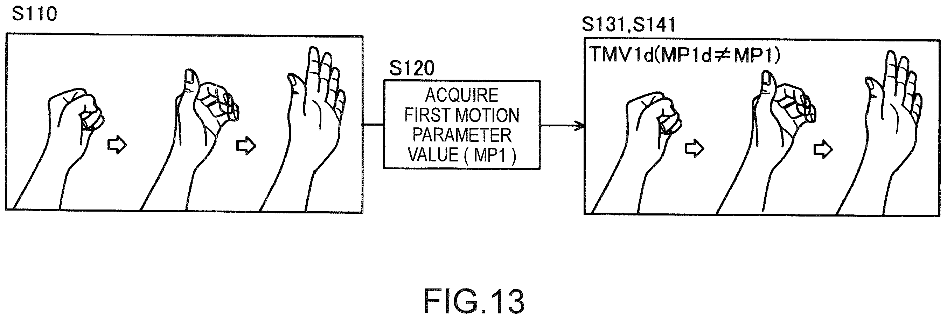

Fig. 12 is a processing flowchart of a training device according to a second embodiment. Fig. 13 is an explanatory view schematically illustrating some of the processing steps of the processing flowchart according to the second embodiment. A configuration of a training device 100 according to the second embodiment has the same as the configuration of the training device 100 according to the first embodiment. The second embodiment is different from the first embodiment in that steps S131 and S141 are provided instead of steps S130 and S140 of Fig. 3. The other operations are the same.

In step S131, the training image generator 230 generates a training image TMV1d having a motion parameter value different from the first motion parameter value MP1, based on the first motion parameter value MP1, and stores the generated training image in the memory 240. In step S141, the controller 200 selects the training image TMV1d as the first training image TMV1 and transmits the training image to the head mounted display 110.

According to the second embodiment, the first motion parameter value MP1 is acquired based on a first motion of the user 300, one training image TMV1d including a training image having a motion parameter value representing motion characteristics different from the first motion parameter value MP1 is generated, and the training image TMVd1 is displayed as the first training image TMV1, and thereby, the user 300 can do motion training while viewing the image 331 of the appropriate first training image TMV1. In addition, since the user 300 can view both the image 331 of the first training image TMV1 and his/her motion when doing the motion training, it is possible to obtain sufficient training effects by visual stimulation and to do sufficient motion training.

### Third Embodiment

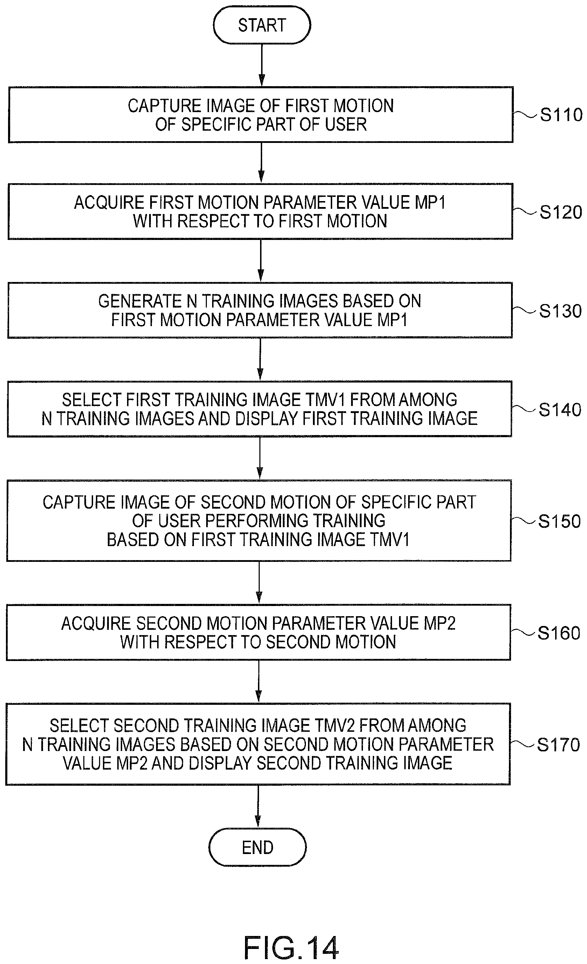

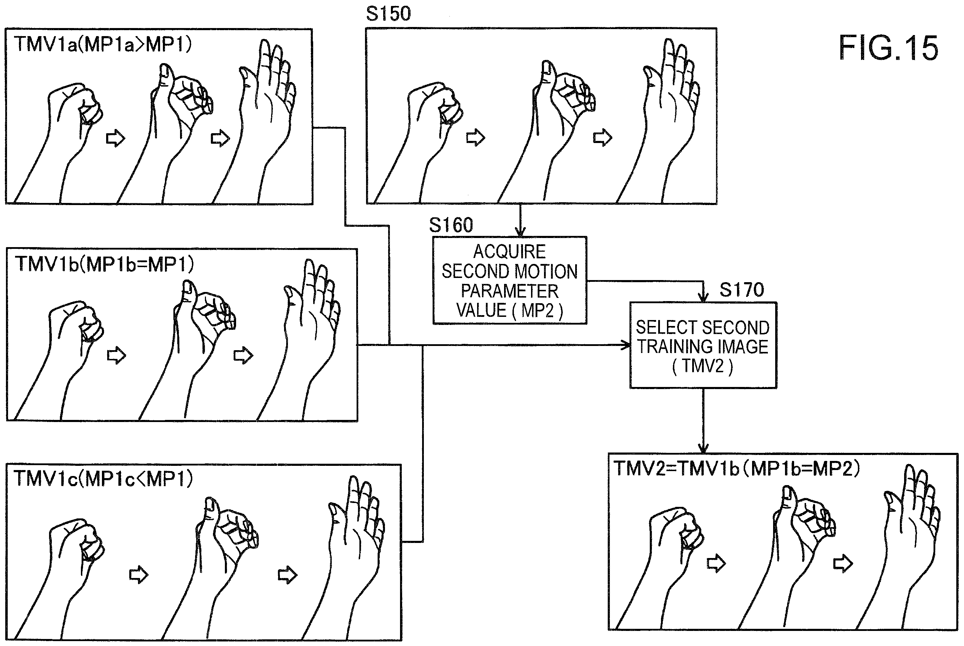

Fig. 14 is a processing flowchart of a training device 100 according to a third embodiment. Fig. 15 is an explanatory view schematically illustrating some of a second half of the processing steps of the processing flowchart of the training device 100 according to the third embodiment. A configuration of the training device 100 according to the third embodiment is the same as the configuration of the training device 100 according to the first embodiment. This processing flowchart is different in that steps S150, S160, and S170 are further included after step S140 of the processing flowchart illustrated in Fig. 3.

The process of step S150 is the same as the process of step S110, and the image-capturer 150 captures an image of a second motion of the specific part of the user 300. The second motion is a motion when motion training is performed based on the first training image TMV1. The processing of step S160 is the same as the processing of step S120, and the motion parameter value acquirer 220 acquires a second motion parameter value MP2 relating to the second motion. In the third embodiment, the moving speed of the joint is used as the second motion parameter value MP2, in the same manner as the first motion parameter value MP1.

In step S170, the training image generator 230 selects one training image from among the N training images TMV1a, TMV1b, and TMV1c as the second training image TMV2, based on the second motion parameter value MP2, and displays the selected training image. The second training image TMV2 may be the same training image as the first training image TMV1 displayed in step S140, but it is preferable to select a different training image. Thus, in the third embodiment, it is preferable that the number N of training images to be generated in step S130 is greater than or equal to 2. For example, various methods which will be described below can be adopted as the method of selecting the second training image TMV2 from among the N training images TMV1a, TMV1b, and TMV1c. Which method is adopted may be determined by an input to the operation unit 270.
(a) The user 300 or a coach of the user 300 of the training device 100 selects a training image by using the operation unit 270.
(b) A training image is selected according to a predetermined selection rule (second selection rule). A specific example thereof is as follows.
   (b1) A training image having a motion parameter value closest to the second motion parameter value MP2 and larger than the motion parameter value MP2 is selected as the second training image TMV2.
   (b2) A training image having a motion parameter value closest to a value obtained by multiplying the second motion parameter value MP2 by a predetermined coefficient is selected as the second training image TMV2. It is preferable to use a value exceeding 1 as the coefficient, for example, a value greater than or equal to 1.1 and less than or equal to 1.3 can be used as the coefficient.
   (b3) The training image is randomly selected from among the N training images.

The present embodiment adopts (b1). In a case where (a) is adopted, for example, if the coach views the second motion parameter value or a state of the motion training of the user 300 and determines that there is a margin for the user 300, the coach trains the user 300 by selecting a training image having a large motion parameter value as the second training image TMV2, and if the coach determines that there is no margin, the coach trains the user 300 by selecting a training image having a small motion parameter value as the second training image TMV2.

As such, according to the third embodiment, the appropriate second training image TMV2 can be selected based on the second motion parameter value MP2 of the second motion of the user 300 who does motion training by viewing the first training image TMV1, and thus, it is possible for the user 300 to do efficient motion training. In addition, since the user 300 can view both the second training image TMV2 and his/her motion when doing the motion training, it is possible to obtain sufficient training effects by visual stimulation.

### Fourth Embodiment

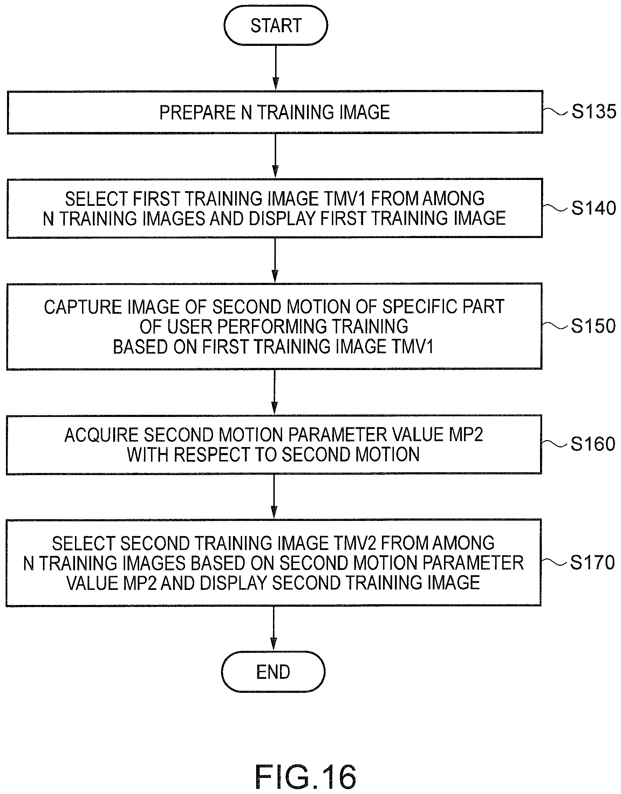

Fig. 16 is a processing flowchart of a training device 100 according to a fourth embodiment. A configuration of the training device 100 according to the fourth embodiment is the same as the configurations of the training devices 100 according to the first and third embodiments. The processing flowchart of the fourth embodiment differs from the processing flowchart according to the third embodiment illustrated in Fig. 14 in that step S135 is provided instead of steps S110, S120, and S130.

In step S135, N (an integer greater than or equal to 2) training images having different motion parameter values are prepared in advance. That is, in the processing flowchart according to the third embodiment illustrated in Fig. 14, the first motion parameter value MP1 is acquired based on the first motion of the user 300, and N training images TMV1a, TMV1b, and TMV1c are generated based on the first motion parameter value MP1. Meanwhile, in the fourth embodiment, a point is different in that the first motion parameter value MP1 is not acquired and N training images TMV1a, TMV1b, and TMV1c having different motion parameter values are prepared in advance.

In step S140, for example, the following various methods can be adopted as a method of selecting the first training image from among the N training images TMV1a, TMV1b, and TMV1c.
(a) The user 300 or a coach of the user 300 of the training device 100 selects a training image by using the operation unit 270.
(b) A training image is selected according to a predetermined selection rule (third selection rule). A specific example thereof is as follows.
   (b1) A training image of which motion parameter value is a median value among the N training images is selected.
   (b2) The training image is randomly selected from among the N training images.

Motions after step S150 are the same as the processing flowchart illustrated in Fig. 14.

As such, also in the fourth embodiment, the appropriate second training image TMV2 is selected based on the second motion parameter value MP2 of the second motion of the user 300 who does the motion training, and causes the user 300 to do the motion training, and thus, it is possible for the user to do an efficient motion training. In addition, since the user 300 can view both the second training image TMV2 and his/her motion when doing the motion training, it is possible to obtain sufficient training effects by visual stimulation.

### Fifth embodiment

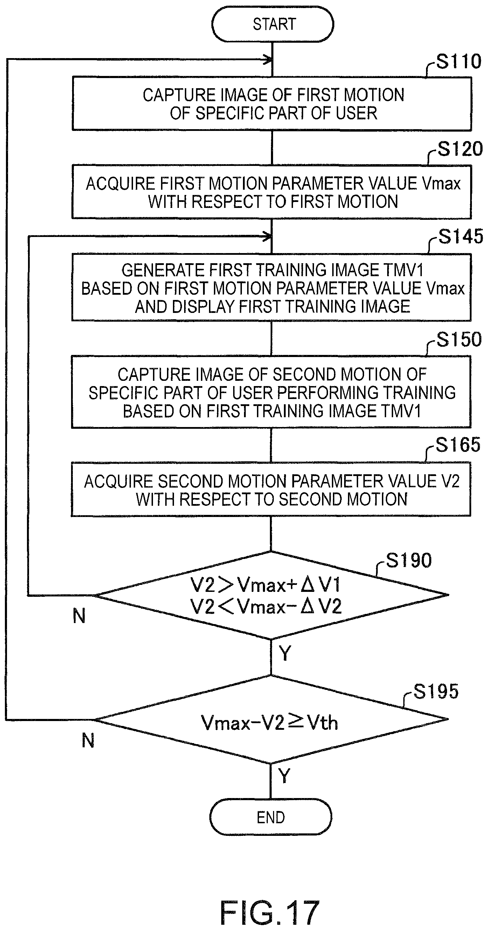

Fig. 17 is a processing flowchart of a training device 100 according to a fifth embodiment. A configuration of the training device 100 according to the fifth embodiment is the same as the configurations of the training devices 100 according to the first to fourth embodiments. As compared with the processing flowchart according to the third embodiment illustrated in Fig. 14, the processing flowchart illustrated in Fig. 17 is different from the processing flowchart according to the third embodiment in that steps S130, S140, S160, and S170 are not included and steps S145, S165, S190, and S195 are included.

As described above, in step S120, the motion parameter value acquirer 220 acquires the first motion parameter value relating to the first motion of the specific part of the user 300. In the fifth embodiment, the maximum speed Vmax of the moving speed of a joint which does a plurality of motions is used as the first motion parameter value. In step S145, the training image generator 230 generates the first training image TMV1 based on the first motion parameter value Vmax and displays the generated image. The first training image TMV1 has a motion parameter value obtained by multiplying the first motion parameter value Vmax by a coefficient greater than 1, for example, a motion parameter value which is 1. 2 times the first motion parameter value.

In step S165, the motion parameter value acquirer 220 acquires the second motion parameter value relating to the second motion of the specific part of the user 300. Moving speed V2 (simply referred to as "speed V2") of the joint of the specific part is used as the second motion parameter value. In step S190, it is determined whether or not a second motion parameter value V2 deviates from a range defined for the first motion parameter value Vmax. The range is a range of Vmax - ΔV2 to Vmax + ΔV1, and ΔV1 and ΔV2 are predetermined values. In a case where the second motion parameter value V2 does not deviate from a predetermined range, that is, in a case where the second motion parameter value V2 is within the predetermined range, the processing returns to step S145, and the user 300 repeats the motion training. However, in a case where the processing returns from step S190 to step S145, the training device 100 does not perform regeneration of the first training image TMV1 and uses the first training image TMV1 generated in step S145 of the previous routine as it is. Meanwhile, in a case where the second motion parameter value V2 deviates from the predetermined range, the processing proceeds to step S195. In step S195, in a case where the amount of attenuation (Vmax - V2) of the second motion parameter value V2 with respect to the first motion parameter value is larger than or equal to a predetermined threshold value Vth, the motion training ends, and in a case where the amount of attenuation is less than the predetermined threshold value Vth, the processing returns to step S110. In a case where the amount of attenuation (Vmax - V2) of the second motion parameter value V2 with respect to the first motion parameter value is large, for example, it is considered that a motion of the user 300 is delayed due to fatigue, therefore, it is preferable to stop the motion training.

As such, according to the fifth embodiment, the user 300 can do motion training by using the training image TMV1 having a motion parameter value greater than the first motion parameter value Vmax of the first motion, and thus, it is possible for the user to do efficient motion training. In addition, since the user 300 can view both the image 331 (for example, Fig. 6 and the like) of the training image TMV1 and his/her motion when doing a motion training, it is possible for the user 300 to obtain sufficient training effects by visual stimulation.

### Modification Example 1

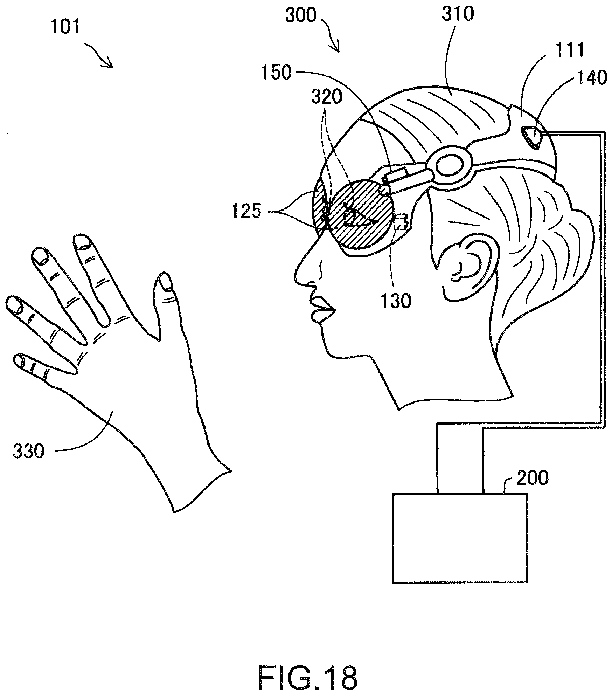

Fig. 18 is an explanatory view illustrating a training device 101 according to Modification Example 1. The training device 100 according to the first to fifth embodiments has the transmissive head mounted display 110 as the head mounted display, whereas, in a training device 101 according to Modification Example 1, an immersion type (also referred to as a "shield type" or a "non-transmissive type") head mounted display 111 is provided as the head mounted display. That is, the display 125 facing the eyes 320 of the user 300 is an immersion display, and displays an image of a body motion of the user 300 captured by the image-capturer 150 in real time, together with the first training image. The display 125 does not transmit light from the outside.

According to Modification Example 1, the user 300 views the image of the body motion of the user 300 captured by the image-capturer 150 in real time, together with the first training image or the second training image displayed on the display 125, and thus, it is possible to obtain sufficient training effects by visual stimulation.

### Modification Example 2

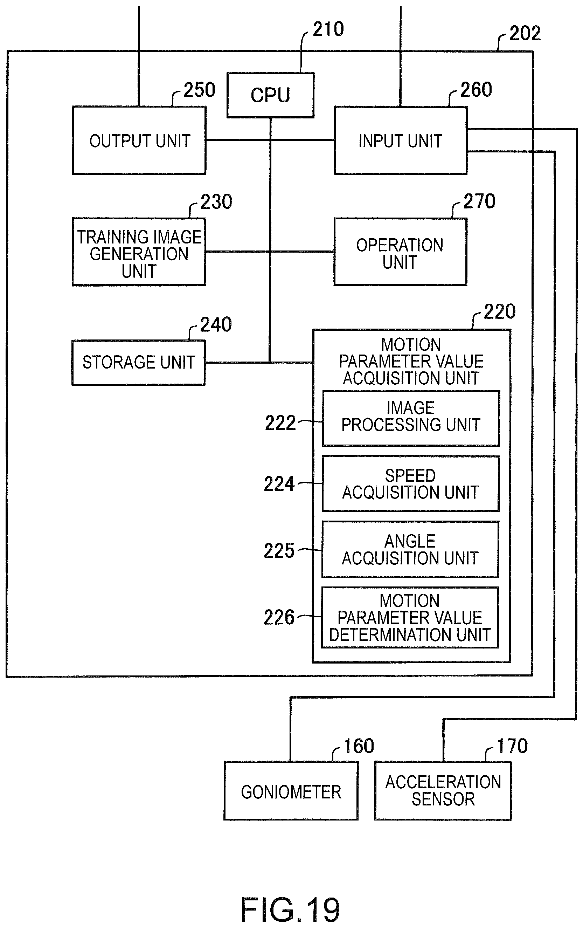

Fig. 19 is an explanatory diagram illustrating the controller 202 according to Modification Example 2. The controller 202 according to Modification Example 2 is different from the controller 200 illustrated in Fig. 2 in that a goniometer 160 and an acceleration sensor 170 are connected to the receiver 260. The goniometer 160 is attached to a joint of a specific part of the user 300 and measures an angle of the joint. The acceleration sensor 170 is attached to the joint of the specific part of the user 300 and measures acceleration when the user 300 moves the joint. The speed acquirer 224 acquires an angle of the joint obtained from the goniometer 160 and can acquire moving speed (or angular speed) from a change rate thereof. In addition, the speed acquirer 224 can acquire the moving speed from the acceleration obtained by the acceleration sensor 170 when the user 300 moves the joint. The angle acquirer 225 acquires a moving angle of the joint of the specific part of the user 300 from an image obtained from the image-capturer 150 or from a signal of the goniometer 160.

The motion parameter value determiner 226 can determine the maximum value of the moving angle of the joint as the motion parameter value. That is, the motion parameter value determiner 226 may use the moving speed of the joint described in the first to fifth embodiments, or may use the moving angle of the joint, as the motion parameter value. For example, (a) both the first motion parameter and the second motion parameter may be used as the moving speed of the specific joint, (b) both the first motion parameter and the second motion parameter may be used as the moving angle of the specific joint, (c) the first motion parameter may be used as one of the moving speed and the moving angle of the specific joint, and the second motion parameter may be used as the other of the moving speed and the moving angle of the specific joint. Even if the motion parameter value is set as the moving angle, the user 300 can do motion training by using the appropriate first training image TMV1 or second training image TMV2. In addition, since the user 300 can view either the first training image TMV1 or the second training image TMV2 and his/her motion when doing the motion training, it is possible to obtain sufficient training effects by visual stimulation.

### Modification Example 3

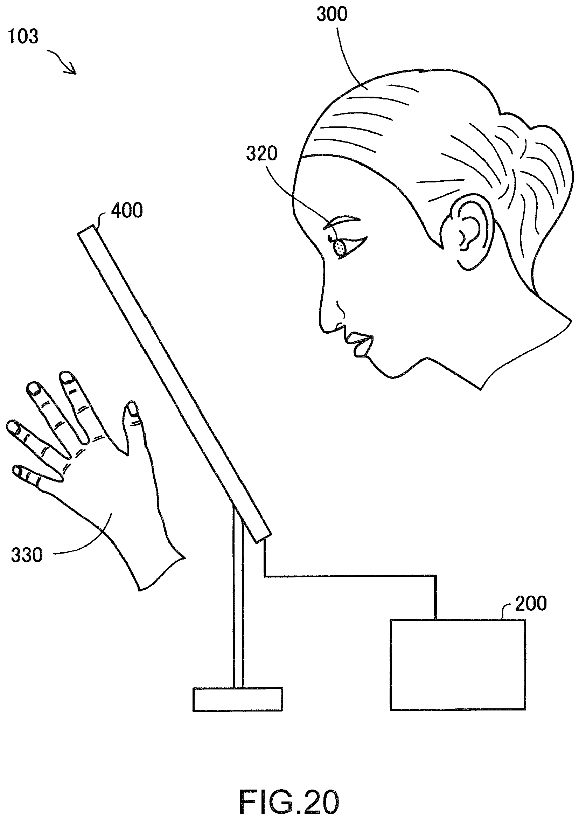

Fig. 20 is an explanatory view illustrating a training device 103 according to Modification Example 3. In the first to fifth embodiments, the display 120 of the head mounted display 110 is used as a display device for displaying a training image, but in the training device 103 according to Modification Example 3, a display device 400 which is not attached to the head 310 is provided instead of the head mounted display 110. The display device 400 displays the training image supplied from the controller 200. In addition, the display device 400 is a transmissive display device, and the user 300 can view an area behind the display device 400. That is, as the user 300 disposes the display device 400 between the eye 320 and the hand 330 which is a specific part, actually, as the user 300 puts the hand 330 on a rear side of the display device 400 as viewed from the user 300, the user can view both the training image and the hand 330 which is a specific part through the display device 400.

### Modification Example 4

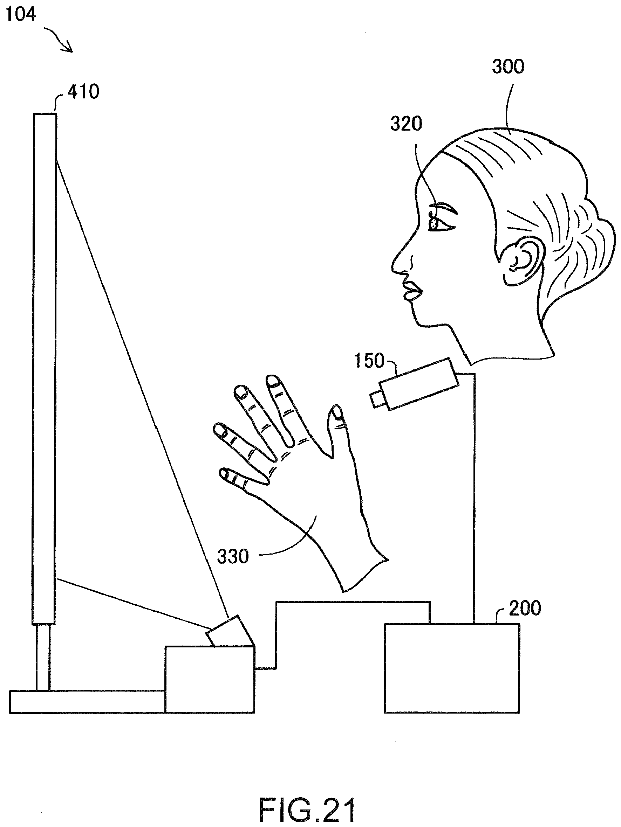

Fig. 21 is an explanatory view illustrating a training device 104 according to Modification Example 4. In the first to fifth embodiments, the display 120 of the head mounted display 110 is used as a display device for displaying a training image, but in the training device 104 according to Modification Example 4, a display device 410 which is not attached to the head 310 is provided instead of the head mounted display 110. The display device 410 simultaneously displays both a training image and an image captured by the image-capturer 150. Various display devices such as a CRT, a liquid crystal display, a plasma display, an organic EL, or a project can be used as the display device 410. As illustrated in Modification Examples 3 and 4, the display device is not limited to the head mounted display 110, and various display devices can be used.

### Modification Example 5

In the above-described embodiments and Modification Examples, training in which the hand 330 is opened from a state of being grasped as the motion training is described as an example, but the embodiments and Modification Examples can also be applied to training in which the hand 330 is grasped from a state of being opened. In addition, motion training of one hand 330 is described as an example, but it is also possible to simultaneously train both hands 330 and 340. In this case, training images of both hands 330 and 340 may be used as training images for different motions. Furthermore, the embodiments and Modification Examples can also be used for motion training of other body parts other than hands, for example, other joints such as knees and elbows.

While embodiments of the invention have been described based on several examples, the embodiments of the invention described above are for easy understanding of the invention, and do not limit the invention. The invention can be changed and improved without departing from the spirit and the scope of the appended claims, and equivalents are included in the invention.

## Claims

1. A rehabilitation training device, comprising:
a motion parameter value acquirer configured to acquire a first motion parameter value relating to a first motion of a body of a user using the training device;
a training image generator configured to generate a training image having a motion parameter value representing motion characteristics different from the first motion parameter value with respect to the first motion, based on the first motion parameter value; and
a display configured to display the training image.

2. The training device according to Claim 1,
wherein the training image generator is configured to generate N training images including the training image having the motion parameter value representing the motion characteristics different from the first motion parameter value with respect to the first motion, based on the first motion parameter value,
wherein the display is configured to display a first training image that is selected from the N training images, and
wherein N is an integer greater than or equal to 2.

3. The training device according to Claim 2, wherein the display is a transmissive display.

4. The training device according to Claim 2, further comprising:
an image-capturer that captures an image of a body motion of the user,
wherein the display is an immersion display.

5. The training device according to any one of claims 2 to 4,
wherein the motion parameter value acquirer is configured to acquire a second motion parameter value relating to a second motion when the user does motion training, based on the first training image,
wherein the training image generator is configured to select a second training image from among the N training images, based on the second motion parameter value, and
wherein the display is configured to display the second training image.

6. The training device according to Claim 5, wherein the first motion parameter value and the second motion parameter value are moving speeds of a specific joint of the user.

7. The training device according to Claim 5, wherein the first motion parameter value is one of a moving speed and a moving angle of a specific joint of the user, and the second motion parameter value is the other one of the moving speed and the moving angle of the specific joint.

8. The training device according to any one of claims 2 to 7, further comprising:
at least one of an image-capturer configured to capture an image of a body of the user;
a goniometer configured to measure an angle of a specific joint of the user; and
an acceleration sensor configured to measure acceleration when the specific joint moves,
wherein the motion parameter value acquirer is configured to acquire the first motion parameter value and the second motion parameter value by using at least one of an output of the image-capturer, an output of the goniometer, and an output of the acceleration sensor.

9. A controller of a rehabilitation training device comprising:
a motion parameter value acquirer configured to acquire a first motion parameter value relating to a first motion of a body of a user using the training device;
a training image generator configured to generate N training images including a training image having a motion parameter value representing motion characteristics different from the first motion parameter value with respect to the first motion, based on the first motion parameter value; and
a transmitter that outputs one training image of the N training images to a display as a first training image;
wherein N is an integer greater than or equal to 2.

10. A rehabilitation training device, comprising:
a memory configured to store in advance N training images having motion parameter values different from each other with respect to a first motion of a body of a user using the training device, among N training images; and
a display that displays a first training image which is selected from the N training images
wherein N is an integer greater than or equal to 2.

11. A computer program of a training device usable for rehabilitation, causing the training device to do,
acquiring a first motion parameter value relating to a first motion of a body of a user who uses the training device;
generating N (an integer greater than or equal to 2) training images including a training image having a motion parameter value representing motion characteristics different from the first motion parameter value with respect to the first motion, based on the first motion parameter value; and
displaying a first training image that is selected from the N training images.

12. A rehabilitation training device, comprising:
a sensor configured to acquire one of a speed and an angle of a moving body part of a user using the training device;
an image generator configured to generate a plurality of training images based on the one of the speed and angle, the training images all having a difference in speed or angle relative to the one of the speed and angle; and
a display configured to selectively display the training images to the user simultaneously with a real or virtual image of the body part.

13. The training device according to Claim 12, wherein the display is a transmissive display.

14. The training device according to Claim 12, wherein the display is an immersion display.
